# EUROPEAN PATENT APPLICATION

(11) **EP 4 368 174 A1**
(43) Date of publication of application: **15.05.2024**
(21) Application number: 22837869.1
(22) Date of filing: 27.06.2022
(51) Int. Cl.: A61K 9/24, A61K 9/20, A61K 31/4985, A61K 31/7034, A61K 31/155, A61P 3/10

(54) **ORAL COMPLEX TABLET COMPRISING SITAGLIPTIN, DAPAGLIFLOZIN, AND METFORMIN**

(30) Priority: 08.07.2021 KR 20210089566
(71) Applicant: Hanmi Pharm. Co., Ltd., Hwaseong-si, Gyeonggi-do 18536 (KR)
(72) Inventor: TAK, Jin Wook, Hwaseong-si, Gyeonggi-do 18536 (KR); BAEK, Ji Won, Hwaseong-si, Gyeonggi-do 18536 (KR); KWON, Taek Kwan, Hwaseong-si, Gyeonggi-do 18536 (KR); IM, Ho Taek, Hwaseong-si, Gyeonggi-do 18536 (KR); KIM, Yong Il, Hwaseong-si, Gyeonggi-do 18536 (KR)
(74) Representative: dompatent von Kreisler Selting Werner - Partnerschaft von Patent- und Rechtsanwälten mbB
(86) International application number: PCT/KR2022/009126
(87) International publication number: WO 2023/282517

(57) **Abstract**

The present invention relates to an oral complex table in the form of a bi-layer tablet, which contains colloidal silicon dioxide in an amount of 0.5 to 2% by weight based on the total weight of the first layer, and a method for manufacturing thereof.

## Description

### [Technical Field]

This application claims the benefit of priority based on Korean Patent Application No. 10-2021-0089566, dated July 8, 2021, and includes all contents disclosed in the document of the Korean Patent Application as part of this specification.

The present invention relates to an oral complex tablet comprising sitagliptin, dapagliflozin and metformin, and a method for manufacturing thereof. More specifically, it relates to an oral complex tablet that has no tabletting difficulties and does not cause layer separation, and a method for manufacturing thereof.

### [Background Art]

Patients with type 2 diabetes are generally accompanied by overweight, abdominal obesity, and high blood pressure. As a result, diabetes is known to be a disease that causes secondary chronic diseases such as high blood pressure, hyperlipidemia, myocardial infarction, and stroke, or metabolic syndrome. According to the Korean Diabetes Association's treatment guidelines, drug combination therapy is actively recommended to increase symptom improvement. In particular, the combination of DPP-4 inhibitor class drugs and SGLT-2 inhibitor class drugs has recently been proven to have excellent efficacy and effectiveness in treating diabetes in academic circles, and even triple treatment with metformin is being studied.

Sitagliptin (product name: Januvia Tablet) is a dipeptidyl peptidase-4 (DPP-4) inhibitor class drug, and the compound name is (R)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one. Sitagliptin regulates blood sugar by inhibiting the breakdown of gastrointestinal hormones called incretins, thereby ensuring that the functions of incretins that regulate insulin and glucagon are carried out in the body. It is known that when sitagliptin is administered orally to patients with type 2 diabetes, HbA1c levels are significantly reduced, and fasting blood sugar and postprandial blood sugar secretion are reduced.

Dapagliflozin (product name: Forxiga Tablet) is a sodium-glucose cotransporter 2 (SGLT-2) inhibitor class drug, and the compound name is (2S,3R,4R,5S,6R)-2-[4-chloro-3-(4-ethoxybenzyl)phenyl]-6-(hydroxymethyl)tetrahydro-2H-pyran-3,4,5-triol. Dapagliflozin can normalize plasma glucose levels by selectively inhibiting SGLT2 in the kidney and enhancing urinary glucose excretion, thereby improving insulin sensitivity and delaying the onset of diabetic complications. AstraZeneca AB, the original developer, is marketing it in tablet form (Forxiga), which contains dapagliflozin propylene glycol hydrate as the active ingredient.

Metformin is a biguanide class diabetes treatment agent and is an oral antihyperglycemic drug mainly used to treat patients with type 2 diabetes. Metformin's blood sugar control mechanism is known to work independently of insulin secretion and, for example, activates the glucose transporter in the liver. Metformin induces weight loss in diabetic patients and has the effect of reducing blood triglycerides and low-density lipoproteins and increasing high-density lipoproteins. Therefore, it can be used as a first-line drug for non-insulin-dependent diabetic patients showing insulin resistance.

Metformin is commercially available in tablet form as its hydrochloride salt, Glucophage (Bristol-Myers Squibb Company). Commercially available Glucophage tablets contain 500 mg, 850 mg, or 1000 mg of metformin hydrochloride, and their administration is within the range of not exceeding the maximum required dose of 2550 mg per day, considering both aspects of efficacy and tolerance. Side effects related to the use of metformin include loss of appetite, abdominal bloating, nausea, and diarrhea, which occur in 20 to 30% of patients taking it. Most of them are transient and disappear after 2 to 3 weeks after taking metformin. It is best to stop taking it if diarrhea or severe abdominal bloating does not go away. In rare cases, skin rash and hives may occur. These side effects can be partially avoided by reducing the minimum and/or sustained dose or using sustained-release formulations that can reduce the frequency of administration.

For diabetes, one or more antidiabetic drugs are often administered in combination for the purpose of controlling blood sugar levels and reducing side effects. In addition to the main effect of sitagliptin and dapagliflozin to lower blood sugar without the risk of hypoglycemia, sitagliptin has a pancreatic beta cell protection effect and GLP-1 increase effect, and dapagliflozin has a weight loss effect and blood pressure lowering effect. Therefore, clinical results show that the combination of the two active ingredients shows a synergistic effect. In addition, when glycemic control is not effective with sitagliptin alone or double administration of sitagliptin and metformin, it has been reported that triple administration of sitagliptin, metformin and dapagliflozin by adding dapagliflozin may be effective in controlling blood sugar (Diabetes Care 2014 Mar; 37(3): 740-750).

In addition, in the case of diabetic patients, as diabetes progresses, blood sugar control becomes more difficult and complications arise. In particular, elderly diabetic patients are more likely to suffer from high blood pressure, obesity, and hyperlipidemia. Due to the characteristics of these diabetic patients, medication compliance is a very important factor, and a decrease in medication compliance not only reduces the patient's quality of life, but also reduces the patient's cure rate, and can lead to an increase in personal medical expenses and a deterioration of insurance finances. Therefore, it is necessary to develop a triple complex tablet containing sitagliptin, dapagliflozin and metformin as the main ingredients.

However, the development of the complex tablet is difficult due to numerous problems such as productivity and stability of the tablet due to differences in the physical properties of each main ingredient (API). For example, metformin requires wet granulation due to problems with flowability during tablet manufacturing, while sitagliptin and dapagliflozin are unstable in moisture. In addition, in the case of dapagliflozin, the density is low and despite a small amount, the volume of the main ingredient is large, so productivity is not good, and there is a high possibility of layer separation from other main ingredients and excipients.

Therefore, there is a need to develop a triple complex tablet containing sitagliptin, dapagliflozin and metformin that can solve the above problems.

### [Prior Art Documents]

### [Non-Patent Document]

Diabetes Care 2014 Mar; 37(3): 740-750

### [Disclosure]

### [Technical Problem]

The present invention is objected to provide an oral complex tablet that has no tabletting difficulties and does not cause separation between layers.

### [Technical Solution]

In order to achieve the above object,
the present invention provides an oral complex tablet, which comprises:
a first layer comprising:
dry granules containing sitagliptin or a pharmaceutically acceptable salt thereof, or a hydrate thereof, and dapagliflozin or a pharmaceutically acceptable salt thereof, or a hydrate thereof, and
colloidal silicon dioxide; and
a second layer comprising wet granules containing metformin or a pharmaceutically acceptable salt thereof,
wherein the colloidal silicon dioxide is contained in an amount of 0.5 to 2% by weight based on the total weight of the first layer.

Further, the present invention provides a method for manufacturing the oral complex tablet of the present invention, which comprises:
(1)a step of preparing a mixture containing: sitagliptin or a pharmaceutically acceptable salt thereof, or a hydrate thereof; dapagliflozin or a pharmaceutically acceptable salt thereof, or a hydrate thereof; and excipients;
(2) a step of dry granulating the mixture;
(3) a step of preparing a fist mixture by mixing the dry granules with colloidal silicon dioxide and a lubricant;
(4) a step of preparing a second mixture by wet granulating a mixture containing metformin or a pharmaceutically acceptable salt thereof, or a hydrate thereof and excipients; and
(5) a step of placing the second mixture in the lower layer of a bi-layer tablet press, placing the first mixture in the upper layer, and then tabletting,
wherein the colloidal silicon dioxide is contained in an amount of 0.5 to 2% by weight based on the total weight of the first mixture.

### [Advantageous Effects]

The oral complex tablet of the present invention comprises both a first layer containing sitagliptin and dapagliflozin and a second layer containing metformin in one preparation, and does not cause tabletting problems such as capping or laminating, and does not cause separation between layers, so the stability of the major ingredient can be secured and productivity is excellent.

### [Description of Drawings]

Fig. 1 is a graph showing the results of measuring the total amount of related compounds of sitagliptin in the complex tablets of Examples 1 to 4 and Comparative Examples 3 to 4.
Fig. 2 is a graph showing the results of measuring the total amount of related compounds of dapagliflozin in the complex tablets of Examples 1 to 4 and Comparative Examples 3 to 4.
Fig. 3 is a graph showing the results of measuring the sitagliptin dissolution rate from the complex tablet according to the content of colloidal silicon dioxide.
Fig. 4 is a graph showing the results of measuring the sitagliptin dissolution rate of the complex tablet according to the preload of the second layer.

### [Best Mode]

Hereinafter, the present invention will be described in more detail.

All technical terms used in this specification, unless otherwise defined, are used with the same meaning as commonly understood by a person skilled in the art. In addition, although preferred methods and samples are described in this specification, similar or equivalent methods are also included in the scope of this specification. In addition, the numerical values described in this specification are considered to include the meaning of "about" even if not specified. The contents of all publications incorporated by reference herein are hereby incorporated by reference in their entirety. As used herein, the term "about" means that the referenced value may vary to some extent. For example, the value can vary by 10%, 5%, 2%, or 1%. For example, "about 5" means including any value between 4.5 and 5.5, between 4.75 and 5.25, or between 4.9 and 5.1, or between 4.95 and 5.05. As used herein, terms such as "have," "may have," "includes," or "may include" indicate the presence of the corresponding feature (e.g., numerical value, or component such as an ingredient) and do not exclude the presence of additional features.

Herein, the term "required tabletting pressure" refers to the tabletting pressure required during tablet manufacturing in order to obtain the desired tablet hardness.

The present invention relates to an oral complex tablet, which comprises:
a first layer comprising:
dry granules containing Sitagliptin or a pharmaceutically acceptable salt thereof, or a hydrate thereof, and Dapagliflozin or a pharmaceutically acceptable salt thereof, or a hydrate thereof, and
colloidal silicon dioxide; and
a second layer comprising wet granules containing Metformin or a pharmaceutically acceptable salt thereof,
wherein the colloidal silicon dioxide is contained in an amount of 0.5 to 2% by weight based on the total weight of the first layer.

The sitagliptin and dapagliflozin, which are the main ingredients of the first layer, include all of their crystal forms, hydrates, co-crystals, solvates, salts, diastereomers or enantiomers.

The metformin, the main ingredient of the second layer, includes all of its crystal forms, co-crystals, solvates, or isomers.

The pharmaceutically acceptable salt thereof refers to any pharmaceutically acceptable salt that can be commonly used in the art.

In one embodiment, the sitagliptin or a pharmaceutically acceptable salt thereof, or a hydrate thereof may be sitagliptin phosphate.

In one embodiment, the dapagliflozin or a pharmaceutically acceptable salt thereof, or a hydrate thereof may be a pharmaceutically acceptable co-crystal of dapagliflozin. In one embodiment, the dapagliflozin or a pharmaceutically acceptable salt thereof may be dapagliflozin L-proline or dapagliflozin propanediol. In one embodiment, the dapagliflozin may be dapagliflozin propanediol.

In one embodiment, the metformin may be metformin hydrochloride or metformin free base. In one embodiment, the metformin may be metformin free base.

In this specification, even if sitagliptin, dapagliflozin, and metformin are mentioned, it should be understood that this refers to all salts, solvates, and isomers of each of the above-mentioned substances.

The oral complex tablet of the present invention comprises a first layer containing dry granules containing sitagliptin and dapagliflozin and colloidal silicon dioxide; and a second layer containing metformin wet granules.

Since sitagliptin and dapagliflozin are relatively vulnerable to moisture compared to metformin, the stability of the main ingredient can be promoted by having a bi-layer tablet structure. In addition, metformin has low flowability, so excellent flowability can be secured by being in the form of wet granules, and high productivity can be secured when manufacturing bi-layer tablets.

The colloidal silicon dioxide of the present invention may be included in an amount of 0.5 to 2% by weight based on the total weight of the first layer.

When tabletting is made according to the minimum tablet size considering the patient's medication compliance and dosage and the weight ratio of the first layer (upper layer) and the second layer (lower layer), the weight ratio of the second layer to the first layer increases. In addition, since the release pattern of the first layer is immediate release and the release pattern of the second layer is sustained release, the properties of the excipients used in the first layer and the second layer are completely different. Therefore, complex tablets consisting of a first layer and a second layer are prone to tabletting disorder.

The tabletting disorder may be capping or laminating.

The capping or laminating may be caused by a large weight ratio of the second layer to the first layer or a high tabletting pressure during tabletting.

Therefore, in the present invention, By including colloidal silicon dioxide in the first layer in an amount of 0.5 to 2% by weight based on the total weight of the first layer, the tabletting pressure can be lowered to provide a complex tablet with an appropriate hardness range of 15 to 25 kp without tabletting disorder.

As a result of the experiment, it was shown that when the colloidal silicon dioxide is included in an amount of more than 0.5% by weight based on the total weight of the first layer, the tabletting pressure during tabletting to ensure that the complex tablet has appropriate hardness can be lowered. If tabletting pressure is high during tabletting, tabletting disorders such as tablet capping or laminating may occur. However, it was confirmed that tabletting pressure can be lowered by including more than 0.5% by weight of colloidal silicon dioxide, making it possible to manufacture oral complex tablets in the form of bi-layer tablets with appropriate hardness without tabletting disorder(see Experimental Example 1).

In addition, as a result of the experiment, when the colloidal silicon dioxide is included in excess of 2% by weight based on the total weight of the first layer, sitagliptin showed an increase in related compounds exceeding or close to the standard at 3 or 4 weeks of harshness, and dapagliflozin showed an increase in related compounds exceeding or close to the standard at 4 weeks of harshness. Additionally, overall, as the amount of the colloidal silicon dioxide increased, the related compounds of sitagliptin and dapagliflozin increased. Therefore, it was evaluated that using the amount of the colloidal silicon dioxide within 2% by weight based on the total weight of the first layer can ensure stability (see Experimental Example 2).

In other words, the oral complex tablet of the present invention contains the colloidal silicon dioxide in the first layer in an amount of 0.5 to 2% by weight based on the total weight of the first layer, so tabletting disorder does not occur. Therefore, productivity is high, and the occurrence of related compounds during storage is maintained within the standard value, enabling chemical stability. Therefore, it was confirmed that the oral complex tablet of the present invention can be manufactured as a complex tablet with excellent productivity and stability.

In one embodiment, The first layer may include one or more excipients selected from the group consisting of diluents, disintegrants, binders, and lubricants.

The diluent may be selected from the group consisting of, for example, anhydrous dicalcium phosphate, D-mannitol, pregelatinized starch, low-substituted hydroxypropyl cellulose (L-HPC), microcrystalline cellulose, sucrose, sorbitol, xylitol, glucose, and any mixtures thereof, but is not limited thereto. In one embodiment, the diluent may be selected from the group consisting of anhydrous dicalcium phosphate, D-mannitol, pregelatinized starch, low-substituted hydroxypropyl cellulose (L-HPC), microcrystalline cellulose, and any combination thereof.

The lubricant may be selected from the group consisting of, for example, glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combination thereof, but is not limited thereto. In one embodiment, the lubricant may be sodium stearyl fumarate.

The disintegrant may be, for example, crospovidone, cross-linked carboxymethylcellulose sodium (Cross-linked CMC Na, C.CMC Na, or croscarmellose sodium), corn starch, carboxymethylcellulose calcium, sodium starch glycolate, low-substituted hydroxypropyl cellulose (L-HPC), and any combination thereof, but is not limited thereto. In one embodiment, the disintegrant may be cross-linked carboxymethylcellulose sodium.

The binder may be selected from the group consisting of, for example, sodium carboxymethylcellulose, ethylcellulose, hydroxyethylcellulose, hydroxypropyl cellulose, hydroxypropylmethyl cellulose, methylcellulose, gelatin, povidone, and any combination thereof, but is not limited thereto. In one embodiment, the binder may be cross-linked sodium carboxymethylcellulose.

In one embodiment, the first layer may include an excipient selected from anhydrous dicalcium phosphate anhydrous, mannitol, hydroxypropyl cellulose, sodium stearyl fumarate, microcrystalline cellulose, pregelatinized starch, crospovidone, cross-linked sodium carboxymethylcellulose, magnesium stearate and any combinations thereof.

In one embodiment, the second layer may include one or more excipients selected from the group consisting of diluents, binders, sustained-release carriers, and lubricants

The diluent may be selected from the group consisting of microcrystalline cellulose, anhydrous calcium hydrogen phosphate, mannitol, sucrose, lactose, sorbitol, xylitol, glucose, hydroxypropyl cellulose, hydroxypropylmethyl cellulose, and any combination thereof, but is not limited thereto.

The binder may be selected from the group consisting of, for example, sodium carboxymethylcellulose, ethylcellulose, hydroxyethylcellulose, hydroxypropyl cellulose, hydroxypropylmethyl cellulose, methylcellulose, gelatin, povidone, and any combination thereof, but is not limited thereto.

The sustained-release carrier may be any sustained-release carrier known in the art. The sustained-release carrier may be selected from the group consisting of, for example, hydroxypropylmethyl cellulose, hydroxyethylcellulose, hydroxypropyl cellulose, methylcellulose, ethylcellulose, polyethylene oxide, guar gum, locust bean gum, xanthan gum, glyceryl distearate, sodium carboxymethylcellulose, polyvinylpyrrolidone, and any combination thereof, but is not limited thereto. In one embodiment, the sustained-release carrier may be a combination of hydroxypropylmethylcellulose 2208, hydroxypropylmethylcellulose 2910, and locust bean gum. The sustained-release carrier may be contained in an amount of 10 to 50 parts by weight, specifically about 20 to 40 parts by weight, based on 100 parts by weight of metformin or a pharmaceutically acceptable salt thereof.

The lubricant may be selected from the group consisting of calcium stearate, colloidal silicon dioxide(fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combination thereof, but is not limited thereto. In one embodiment, the lubricant may be magnesium stearate or colloidal silicon dioxide.

In one embodiment, the second layer may include excipients selected from sodium stearyl fumarate, hydroxypropyl cellulose, locust bean gum, colloidal silicon dioxide, microcrystalline cellulose, mannitol, sucrose, lactose, sorbitol, xylitol, glucose, and any combinations thereof.

In one embodiment, the complex tablet may be an oral complex tablet in which the total content of the related compounds of sitagliptin is 0.2% by weight or less and the total content of the related compounds of dapagliflozin is 2% by weight or less when stored for 4 weeks under harsh conditions at 60°C.

The complex tablet may contain 25 to 100 mg of sitagliptin as sitagliptin free base, 5 to 10 mg of dapagliflozin as free base, and 500 to 1000 mg of metformin as free base per unit dosage form. For example, the complex tablet may contain 500 mg, 750 mg, 850 mg, or 1,000 mg of metformin, 50 mg of sitagliptin, and 5 mg of dapagliflozin as free base per unit dosage form.

The complex tablet can be formed in the form of a bi-layer tablet by compressing dry granules containing sitagliptin and dapagliflozin as a first layer and wet granules containing metformin as a second layer.

The complex tablet may additionally include a film coating layer on its outer surface. The film coating layer may include any film coating agent that exhibit immediate release properties and colorant. The film coating agent includes, but is not limited to, a mixture of hydroxypropyl cellulose (HPC) and hydroxypropylmethyl cellulose (HPMC), or a mixture of polyvinyl alcohol (PVA) and polyethylene glycol (PEG). The colorant includes titanium dioxide, iron oxide, etc., but is not limited thereto. A representative commercially available film coating agent is Opadry^{®}. The film coating layer can serve to mask taste and provide stability to the final complex tablet..

The complex tablet can be used for the treatment of adult patients with type 2 diabetes who are not sufficiently controlled with sitagliptin, dapagliflozin, or metformin alone or in combination of two types, or patients who are already receiving triple combination treatment of sitagliptin, dapagliflozin, and metformin. The complex tablet can be administered once a day, twice a day, three times a day, or four times a day depending on the content of the main ingredient it contains.

Additionally, the present invention relates to a method for manufacturing an oral complex tablet. Specifically, it relates to a method for manufacturing the oral complex tablet, which comprises:
(1)a step of preparing a mixture containing: Sitagliptin or a pharmaceutically acceptable salt thereof, or a hydrate thereof; Dapagliflozin or a pharmaceutically acceptable salt thereof, or a hydrate thereof; and excipients;
(2) a step of dry granulating the mixture;
(3) a step of preparing a fist mixture by mixing the dry granules with colloidal silicon dioxide and a lubricant;
(4) a step of preparing a second mixture by wet granulating a mixture containing metformin or a pharmaceutically acceptable salt thereof, or a hydrate thereof and excipients; and
(5) a step of placing the second mixture in the lower layer of a double-layer tablet press, placing the first mixture in the upper layer, and then tabletting,
wherein the colloidal silicon dioxide is contained in an amount of 0.5 to 2% by weight based on the total weight of the first mixture.

Details of the manufacturing method of the complex tablet can be directly applied to the description of the complex tablet according to one aspect of the present invention.

In the first layer manufacturing step, the dry granulation step of the step (2) may be prepared according to a typical dry granulation method known in the art. In one embodiment, the dry granulation may include forming a compressed object using a roller compactor from a mixture containing the main ingredient, diluent, binder, and lubricant.

In the second layer manufacturing step, the wet granulation step of the step (4) may be prepared according to a typical wet granulation method known in the art.

Each step involved in the manufacturing method of the complex tablet can be performed based on a typical bi-layer tablet-type complex tablet manufacturing process performed in the relevant technical field.

In one embodiment, in the step (5), the tabletting pressure required for tabletting a bi-layer tablet when manufacturing a complex tablet in the form of a bi-layer tablet may be about 2,000 to 3,000 KN.

The oral complex tablet manufacturing method of the present invention may further comprise a step of applying a preload of 1 to 7KN after placing the second mixture in the lower layer in the step (5).

Layer separation may occur in complex tablets in the form of bi-layer tablets, which may be caused by different properties of each excipient used in the first layer and second layer.

In order to prevent the layer separation phenomenon, an attempt was made to reduce the preload on the second layer. However, if the preload is small, a sustained release phenomenon occurs in the first layer, which is an immediate release layer, due to interference between the first and second layers, making it difficult to properly display the desired drug effect of the first layer.

Therefore, in the present invention, after placing the second mixture in the lower layer, the preload was set to 1 to 7 KN to prevent layer separation and sustained release of the immediate release layer due to interlayer interference.

As a result of the experiment, when the preload of the second mixture was 7KN or less, no layer separation occurred under accelerated exposure conditions. However, when the preload exceeded 7KN and accelerated exposure conditions continued for more than 5 days, layer separation of the complex tablet in the form of a bi-layer tablet occurred. Therefore, it was evaluated that when compressing the second mixture into a second layer, the preload of the second mixture is 7KN or less to prevent layer separation (see Experimental Example 4).

Additionally, as a result of the experiment, when the preload of the second mixture was 1KN or more, the initial dissolution rate of sitagliptin was fast and the deviation was small. However, when the preload was less than 1KN, the immediate dissolution of the first layer was affected by the sustained release effect of the second layer, resulting in a decrease in dissolution. Therefore, it was evaluated that when compressing the second mixture into the second layer, the preload of the second mixture is 1KN or more to prevent sustained release in the first layer, which is the immediate release layer, due to interlayer interference (see Experimental Example 5).

That is, it was confirmed that in the oral complex tablet manufacturing method of the present invention, the layer separation in the bi-layer tablet type complex tablet can be prevented and the sustained release phenomenon of the immediate-release layer can be prevented, by setting the preload of the second mixture to 1 to 7 KN.

Hereinafter, the present invention will be described in detail by Examples. However, the following Example specifically illustrates the present invention, and the content of the present invention is not limited by the following Examples.

### Examples 1 to 4 and Comparative Examples 1 to 4. Preparation of complex tablet

Sitagliptin, dapagliflozin, anhydrous dicalcium phosphate, mannitol, hydroxypropyl cellulose EXF type, and sodium stearyl fumarate were sieved through a No. 30 sieve to crush any large lumps and mixed well. This mixture was pressed with a roller compactor to produce slugs. The prepared slug was sieved through a No. 20 sieve to prepare dry granules. Croscarmellose sodium as a disintegrant and colloidal silicon dioxide as an excipient to ensure tabletting properties were sieved through a No. 30 sieve and mixed with the dry granules prepared above to prepare a second mixed powder. Sodium stearyl fumarate was used as a lubricant and mixed with the second mixed powder to prepare the first layer (upper layer).

After sieving metformin, hydroxypropylmethyl cellulose and locust bean gum, wet granules were prepared using a high-speed granulator using water as a binding solvent. The prepared wet granules were dried to meet moisture standards using a fluidized bed dryer and then sized using a sizing machine. Colloidal silicon dioxide was sieved and mixed with the wet granules, and vegetable magnesium stearate was sieved to prepare a second layer (lower layer).

The first layer and the second layer were tabletted with a preload of 4KN using a bi-layer tablet press, and the outer shell was coated after tableting with a hardness of 20kp as the target.

The contents of the components used in manufacturing the complex tablet are shown in Table 1 below.

**[Table 1]**

| | (Unit : mg) | | | | | | | | | |
|---|---|---|---|---|---|---|---|---|---|---|
| | | | Exam 1 | Exam 2 | Exam 3 | Exam 4 | Comp Exam 1 | Comp Exam 2 | Comp Exam 3 | Comp Exam 4 |
| Feature | Process | Colloidal silicon dioxide content based on total weight of first layer | 0.5% | 1.0% | 1.5% | 2.0% | 0% | 0.25 % | 2.5% | 3.0% |
| First layer (Upper layer) | Dry granule | Sitagliptin phosphate monohydrate | 64.3 | | | | | | | |
| | | Dapagliflozin propanediol | 6.2 | | | | | | | |
| | | Anhydrous dicalcium phosphate | 40.0 | | | | | | | |
| | | mannitol | 137. 0 | 135. 5 | 134. 0 | 132. 5 | 138. 5 | 137. 75 | 131. 0 | 129. 5 |
| | | hydroxypropyl cellulose EXF type | 8.0 | | | | | | | |
| | | Sodium stearyl fumarate | 12.0 | | | | | | | |
| | 2^{nd} mixing | Colloidal silicon dioxide | 1.5 | 3.0 | 4.5 | 6.0 | - | 0.75 | 7.5 | 9.0 |
| | | Croscarmellose sodium | 24.0 | | | | | | | |
| | Final mixing | Sodium stearyl fumarate | 7.0 | | | | | | | |
| Second layer (Lower layer) | Wet granule | Metformin hydrochloride | 1000.0 | | | | | | | |
| | | Hydroxypropylmet hyl cellulose 2208 | 270.0 | | | | | | | |
| | | Hydroxypropylmet hyl cellulose 2910 | 8.0 | | | | | | | |
| | | Locust bean gum | 40.0 | | | | | | | |
| | 2^{nd} mixing | Colloidal silicon dioxide | 14.0 | | | | | | | |
| | Final mixing | Vegetable magnesium stearate | 14.0 | | | | | | | |
| Coating | Outer shell coating | Opadry II PVA | 56.8 | | | | | | | |
| | Polishin g coating | Carnauba wax | 0.2 | | | | | | | |

### Experimental Example 1. Characteristic evaluation of complex tablets

The required tabletting pressure, capping ratio, and friability of the complex tablets manufactured in Examples 1 to 4 and Comparative Examples 1 to 4 were evaluated, and the results are shown in Table 2 below.

Generally, in the case of 1-time friability, it is used as an evaluation item to check tabletting disorder in the tableting process, and in the case of 4-time friability, it is used as an evaluation item to ensure stability of tablet properties within the coating machine. The evaluation standard for 1-time friability is within 0.2%, and the evaluation standard for 4-time friability is within 1%.

**[Table 2]**

| | Exam. 1 | Exam. 2 | Exam. 3 | Exam. 4 | Comp. Exam. 1 | Comp. Exam. 2 | Comp. Exam. 3 | Comp. Exam. 4 |
|---|---|---|---|---|---|---|---|---|
| Colloidal silicon dioxide content based on total weight of first layer (wt%) | 0.5% | 1.0% | 1.5% | 2.0% | 0% | 0.25% | 2.5% | 3.0% |
| Required tabletting pressure when tabletting 20kp | 3000 KN | 2600 KN | 2300 KN | 2000 KN | 3400 KN | 3200 KN | 1800 KN | 1600 KN |
| Capping ratio when tabletting | 0 / 20 | 0 / 20 | 0 / 20 | 0 / 20 | 3 / 20 | 1 / 20 | 0 / 20 | 0 / 20 |
| Capping ratio within coating machine | 0 / 20 | 0 / 20 | 0 / 20 | 0 / 20 | 7 / 20 | 4 / 20 | 0 / 20 | 0 / 20 |
| 1-time friability (within 0.2%) | 0.05% | 0.08% | 0.12% | 0.15% | 0.45% | 0.25% | 0.03% | 0.02% |
| 4-time friability (within 1.0%) | 0.26% | 0.37% | 0.58% | 0.76% | 1.58% | 1.12% | 0.13% | 0.09% |

From the results in Table 2 above, the complex tablets of Examples 1 to 4 containing colloidal silicon dioxide in an amount of 0.5 to 2% by weight based on the total weight of the first layer had a appropriate range of required tabletting pressure of 3000KN or less at which capping does not occur when tableting with a target hardness of 20kp. In addition, the 1-time and 4-time friability also showed good results.

However, for the complex tablets of Comparative Examples 1 and 2, which contained less than 0.5% by weight of colloidal silicon dioxide based on the total weight of the first layer, the required tabletting pressure to secure the target hardness of 20kp was measured to be very high. As a result, it was confirmed that capping occurred during tableting or during rotation within the coating machine when coating, and that the friability was poor.

Therefore, it was found that colloidal silicon dioxide should be included at 0.5% by weight or more based on the total weight of the first layer.

### Experimental Example 2. Evaluation of related compound of complex tablet

By evaluating the amount of related compounds of sitagliptin and dapagliflozin for Examples 1 to 4 and Comparative Examples 3 to 4, the stability of the complex tablet over time according to the amount of colloidal silicon dioxide was evaluated. Specifically, for each bi-layer tablet, the total related compounds for sitagliptin and dapagliflozin were measured at 1, 2, and 4 weeks under harsh conditions at 60°C, respectively.

The measurement method of related compounds is as follows.

### Sample preparation

20 tablets manufactured in Example were taken, crushed, and the equivalent amount of 5 tablets was accurately taken and placed in a 250 mL volumetric flask. 60 mL of acetonitrile and a magnetic bar were added thereto and stirred for 30 minutes. Afterwards, 140 mL of diluted solution was added and stirred for an additional 60 minutes. The magnetic bar was taken out and washed with 10 mL of diluent, then the washed liquid was placed in the corresponding volumetric flask and 25 mL of diluent was added. After adjusting to the marked line with acetonitrile, the solution was mixed well, and this solution was centrifuged at 3000 rpm for 10 minutes. The supernatant was used as the sample solution. The sample solution was tested under the following conditions.

### Mobile phase

Solution A: 0.1% phosphoric acid
Solution B: 85% phosphoric acid : acetonitrile : methanol = 0.1 : 80 : 20 (v/v/v)

### Diluent

pH 2.5 phosphate buffer

### HPLC condition

Column : column filled with 3um octadecyl silylated silica gel in a stainless steel tube with an inner diameter of 4.6 mm and a length of 150 mm.
Pump : 1.2 mL/min
Injection Vol.: 20uL
UV lamp: 220 nm
Analysis time: 80 min

**[Table 3]**

| Time (min) | Volume ratio (Solution A:Solution B) |
|---|---|
| 0 | 80:20 |
| 5 | 80:20 |
| 60 | 40:60 |
| 65 | 80:20 |
| 80 | 80:20 |

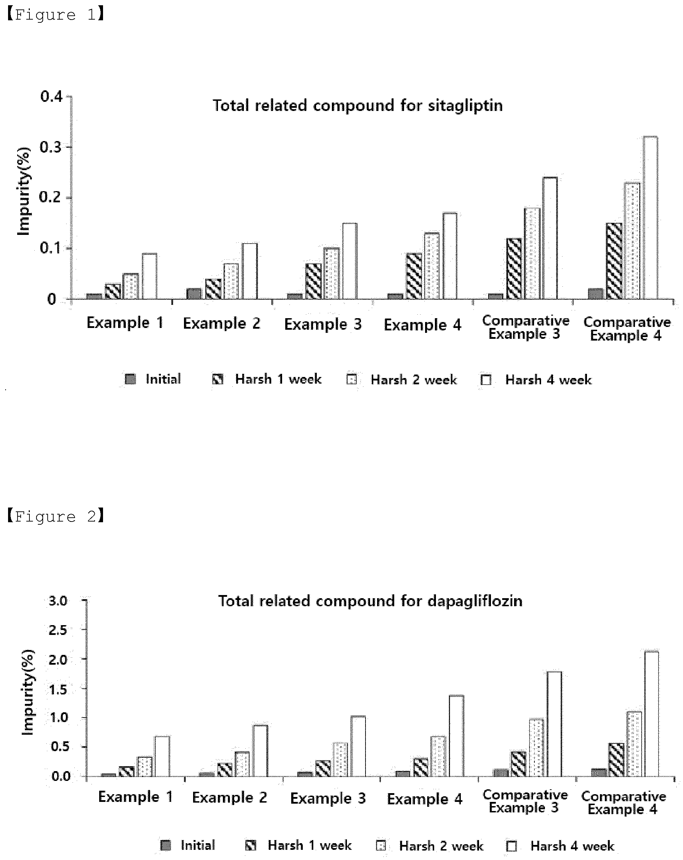

The related compound results of sitagliptin are shown in Table 4 and Fig. 1, and the related compound results of dapagliflozin are shown in Table 5 and Fig. 2.

**[Table 4]**

| | Exam. 1 | Exam. 2 | Exam. 3 | Exam. 4 | Comp. Exam. 3 | Comp. Exam. 4 |
|---|---|---|---|---|---|---|
| Colloidal silicon dioxide content based on total weight of first layer (wt%) | 0.5% | 1.0% | 1.5% | 2.0% | 2.5% | 3.0% |
| Initial | 0.01% | 0.02% | 0.01% | 0.01% | 0.01% | 0.02% |
| Harsh 1 week | 0.03% | 0.04% | 0.07% | 0.09% | 0.12% | 0.15% |
| Harsh 2 week | 0.05% | 0.07% | 0.10% | 0.13% | 0.18% | 0.23% |
| Harsh 4 week | 0.09% | 0.11% | 0.15% | 0.17% | 0.24% | 0.32% |

**[Table 5]**

| | Exam. 1 | Exam. 2 | Exam. 3 | Exam. 4 | Comp. Exam. 3 | Comp. Exam. 4 |
|---|---|---|---|---|---|---|
| Colloidal silicon dioxide content based on total weight of first layer (wt%) | 0.5% | 1.0% | 1.5% | 2.0% | 2.5% | 3.0% |
| Initial | 0.05% | 0.06% | 0.07% | 0.09% | 0.11% | 0.13% |
| Harsh 1 week | 0.16% | 0.23% | 0.27% | 0.31% | 0.42% | 0.55% |
| Harsh 2 week | 0.34% | 0.42% | 0.57% | 0.68% | 0.97% | 1.10% |
| Harsh 4 week | 0.68% | 0.87% | 1.01% | 1.38% | 1.78% | 2.12% |

The stability of related compounds was set at within 0.2% for sitagliptin and within 2% for dapagliflozin by applying the standards of the United States Pharmacopoeia (USP) and existing commercially available single agents under harsh conditions at 60°C.

From the results in Table 4 above, the complex tablets of Examples 1 to 4 showed an increase in the related compound of sitagliptin up to harsh 4 week, but all were within the standard. On the other hand, Comparative Example 3 showed results exceeding the standards for related compounds at harsh 4 week, and Comparative Example 4 showed results exceeding the standards for related compounds from harsh 2 week.

In addition, from the results in Table 5 above, the complex tablets of Examples 1 to 4 showed an increase in the related compound of dapagliflozin up to harsh 4 week, but all were within the standard. On the other hand, Comparative Example 4 showed results exceeding the standards for related compounds in harsh 4 week.

In other words, as the content of colloidal silicon dioxide based on the total weight of the first layer increased, the related compound tended to increase.

From the above results, it was confirmed that the content of colloidal silicon dioxide based on the total weight of the first layer should be less than 2% by weight.

### Experimental Example 3. Sitagliptin dissolution evaluation of complex tablets according to colloidal silicon dioxide content

Sitagliptin dissolution evaluation of the complex tablets of Examples 1 to 4 and Comparative Examples 3 to 4 was performed. The dissolution evaluation was performed at 125 rpm using the Bakset method in 900 mL of solvent at pH 1.2.

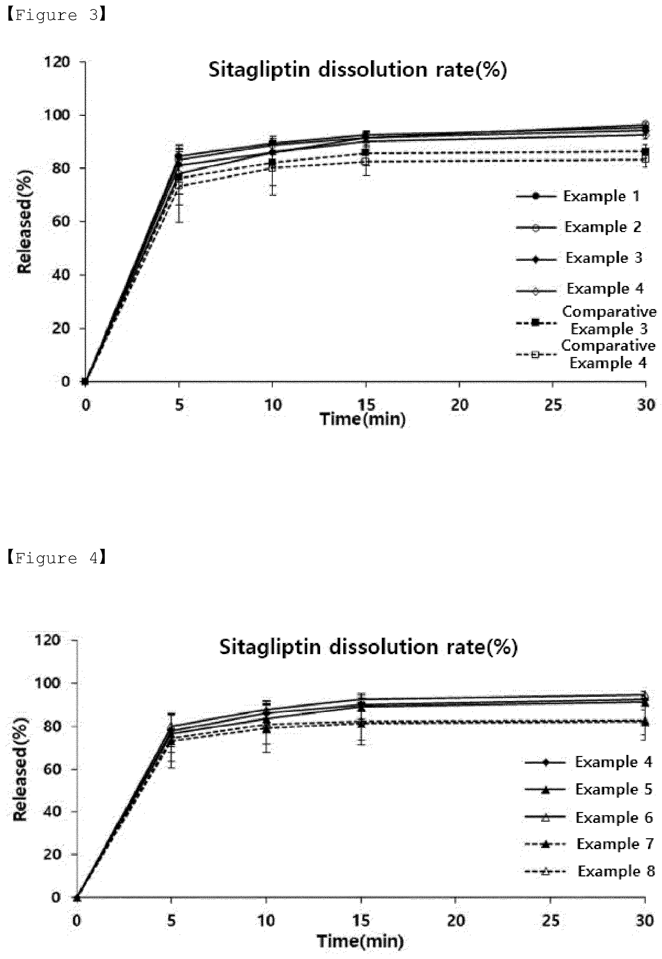

The results are shown in Table 6 below and Fig. 3.

**[Table 6]**

| | | Exam. 1 | Exam. 2 | Exam. 3 | Exam. 4 | Comp. Exam. 3 | Comp. Exam. 4 |
|---|---|---|---|---|---|---|---|
| Colloidal silicon dioxide content based on total weight of first layer (wt%) | | 0.5% | 1.0% | 1.5% | 2.0% | 2.5% | 3.0% |
| 5 min | dissolution rate (%) | 84.6 | 83.2 | 81.2 | 78.2 | 76.5 | 73.4 |
| | deviation | 4.2 | 5.3 | 6.4 | 7.8 | 10.2 | 13.7 |
| 10 min | dissolution rate (%) | 89.5 | 88.7 | 86.1 | 85.9 | 82.1 | 80.0 |
| | deviation | 2.5 | 3.5 | 4.3 | 5.5 | 8.7 | 9.9 |
| 15 min | dissolution rate (%) | 92.4 | 91.6 | 91.4 | 90.1 | 85.7 | 82.5 |
| | deviation | 1.2 | 2.1 | 2.8 | 3.2 | 4.7 | 5.4 |
| 30 min | dissolution rate (%) | 95.3 | 96.4 | 94.2 | 92.5 | 86.2 | 83.4 |
| | deviation | 0.5 | 0.9 | 1.1 | 1.5 | 2.6 | 3.2 |

From the results in Table 6 above, the complex tablets of Examples 1 to 4 showed a rapid disintegration time of the granules and a small deviation in the initial dissolution. On the other hand, the complex tablets of Comparative Examples 3 and 4 showed large deviations in the disintegration time of the granules, large lumps, and large deviations in the initial dissolution.

This is because the granular coating of colloidal silicon dioxide reduces disintegration and increases dissolution deviation. It was found that this decrease in granule disintegration rate was due to the gelling of the matrix of the second layer containing metformin, which is a sustained-release layer, and the immediate dissolution of the first layer was affected by the sustained-release effect of the second layer, resulting in a decrease in dissolution.

Therefore, from the results of Experimental Example 1 to 3 above, It can be seen that when colloidal silicon dioxide is included at 0.5 to 2% by weight of the total weight of the first layer, tabletting disorder does not occur, related compounds occur within the standard, and sustained release of the immediate release layer due to interference between layers can be prevented.

### Experimental Example 4. Layer separation evaluation of complex tablet according to second layer preload

The complex tablets of Examples 5 to 10 were manufactured in the same manner as Example 4 above, but by changing the preload conditions to the conditions in Table 7 below.

It was observed whether layer separation occurred when the complex tablets of Examples 4 to 10 were stored under accelerated exposure conditions for 3, 5, and 7 days, and the results are shown in Table 7 below.

Here, accelerated exposure conditions refer to the conditions of storage in an unsealed Petri dish under temperature and humidity conditions of 40°C and 75%RH.

**[Table 7]**

| Number of layer separations for first layer/second layer under accelerated exposure condition | Exam. 4 | Exam. 5 | Exam. 6 | Exam. 7 | Exam. 8 | Exam. 9 | Exam. 10 |
|---|---|---|---|---|---|---|---|
| second layer preload | 4 KN | 1KN | 7KN | 0.1KN | 0.5KN | 8KN | 9KN |
| 3 day | 0 / 20 | 0 / 20 | 0 / 20 | 0 / 20 | 0 / 20 | 0 / 20 | 1 / 20 |
| 5 day | 0 / 20 | 0 / 20 | 0 / 20 | 0 / 20 | 0 / 20 | 1 / 20 | 2 / 20 |
| 7 day | 0 / 20 | 0 / 20 | 0 / 20 | 0 / 20 | 0 / 20 | 2 / 20 | 4 / 20 |

From the results in Table 7 above, layer separation did not occur in the complex tablets of Examples 4 to 8 where the preload of the second layer (lower layer) was 7KN or less.

However, in the complex tablets of Examples 9 and 10, where the preload of the second layer exceeded 7KN, layer separation was observed to occur from 5 and 3 days, respectively.

If the preload of the second layer, which is the lower layer, exceeds 7KN, the lower layer is pressed into tablet form rather than being compacted while holding the shape. As a result, due to the completely different properties of the excipients of the first layer (upper layer) and the second layer (lower layer), it is the same as compressing two hard tablets into an upper layer and a lower layer. Therefore, the bonding force between the two layers decreases, causing layer separation.

From the above results, it was found that the preload of the second layer should be less than 7KN.

### Experimental Example 5. sitagliptin dissolution evaluation of complex tablet according to second layer preload

Sitagliptin dissolution evaluation of the complex tablets of Examples 4 to 8 was performed. The dissolution evaluation was performed at 125 rpm using the Bakset method in 900 mL of solvent at pH 1.2.

The results are shown in Table 8 below and Fig. 4.

**[Table 8]**

| Sitagliptin dissolution rate (%) | | Exam. 4 | Exam. 5 | Exam. 6 | Exam. 7 | Exam. 8 |
|---|---|---|---|---|---|---|
| lower layer preload | | 4 KN | 1KN | 7KN | 0.1KN | 0.5KN |
| 5 min | Dissolution rate | 78.2 | 76.5 | 80.3 | 73.2 | 74.5 |
| | Deviation | 7.8 | 8.4 | 5.6 | 12.4 | 10.9 |
| 10 min | Dissolution rate | 85.9 | 83.4 | 87.8 | 79.1 | 81.1 |
| | Deviation | 5.5 | 6.4 | 4.2 | 11.2 | 9.4 |
| 15 min | Dissolution rate | 90.1 | 88.9 | 92.4 | 81.2 | 82.3 |
| | Deviation | 3.2 | 5.7 | 2.7 | 9.8 | 8.7 |
| 30 min | Dissolution rate | 92.5 | 91.2 | 94.7 | 82.1 | 82.7 |
| | Deviation | 1.5 | 3.8 | 1.4 | 8.7 | 6.8 |

From the results in Table 8 above, the complex tablets in Examples 4 to 6 showed a fast dissolution rate and a small deviation in initial dissolution. On the other hand, in the complex tablets of Examples 7 and 8, interference occurs between the first layer (upper layer) and second layer (lower layer) granules during compression. As a result, it was found that due to gelling of the matrix of the second layer containing metformin, which is a sustained-release layer, the immediate dissolution of the first layer was affected by the sustained-release effect of the second layer, resulting in a decrease in dissolution.

Therefore, from the results of Experimental Examples 4 and 5, it can be seen that setting the preload of the second layer to 1 to 7KN prevents layer separation of the complex tablet in the form of a bi-layer tablet and prevents the sustained release phenomenon of the immediate release layer due to interlayer interference.

## Claims

1. An oral complex tablet, comprising:
a first layer comprising:
dry granules containing sitagliptin or a pharmaceutically acceptable salt thereof, or a hydrate thereof, and dapagliflozin or a pharmaceutically acceptable salt thereof, or a hydrate thereof, and
colloidal silicon dioxide; and
a second layer comprising wet granules containing metformin or a pharmaceutically acceptable salt thereof,
wherein the colloidal silicon dioxide is contained in an amount of 0.5 to 2% by weight based on the total weight of the first layer.

2. The oral complex tablet according to claim 1, wherein the fist layer contains sitagliptin phosphate and dapagliflozin propanediol as a major ingredient.

3. The oral complex tablet according to claim 1, wherein the second layer contains metformin free base as a major ingredient.

4. The oral complex tablet according to claim 1, wherein the first layer comprises excipients selected from anhydrous dicalcium phosphate, mannitol, hydroxypropyl cellulose, sodium stearyl fumarate, microcrystalline cellulose, pregelatinized starch, crospovidone, cross-linked sodium carboxymethylcellulose, magnesium stearate and any combinations thereof.

5. The oral complex tablet according to claim 1, wherein the second layer comprises excipients selected from sodium stearyl fumarate, hydroxypropyl cellulose, locust bean gum, colloidal silicon dioxide, microcrystalline cellulose, mannitol, sucrose, lactose, sorbitol, xylitol, glucose and any combinations thereof.

6. The oral complex tablet according to claim 1, wherein when the complex tablet is stored under harsh conditions at 60°C for 4 weeks, the total content of related compounds of sitagliptin is less than 0.2% by weight, and the total content of related compounds of dapagliflozin is less than 2% by weight.

7. The oral complex tablet according to claim 1, wherein each unit formulation contains 25 to 100 mg of sitagliptin as sitagliptin free base, 5 to 10 mg of dapagliflozin as dapagliflozin free base, and 500 to 1000 mg of metformin as metformin free base.

8. A method for manufacturing the oral complex tablet according to any one of claim 1 to claim 7, which comprises the steps of:
(1) preparing a mixture containing: sitagliptin or a pharmaceutically acceptable salt thereof, or a hydrate thereof; dapagliflozin or a pharmaceutically acceptable salt thereof, or a hydrate thereof; and excipients;
(2) dry granulating the mixture;
(3) preparing a fist mixture by mixing the dry granules with colloidal silicon dioxide and a lubricant;
(4) preparing a second mixture by wet granulating a mixture containing metformin or a pharmaceutically acceptable salt thereof, or a hydrate thereof and excipients; and
(5) placing the second mixture in the lower layer of a bi-layer tablet press, placing the first mixture in the upper layer, and then tabletting,
wherein the colloidal silicon dioxide is contained in an amount of 0.5 to 2% by weight based on the total weight of the first mixture.

9. The method for manufacturing the oral complex tablet according to claim 8, wherein the dry granulation of the step (2) comprises a step of forming a compressed object using a roller compactor.

10. The method for manufacturing the oral complex tablet according to claim 8, which further comprises a step of applying a preload of 1 to 7KN after placing the second mixture in the lower layer in the step (5).

11. The method for manufacturing the oral complex tablet according to claim 8, wherein the required tabletting pressure in the step (5) is 2,000 to 3,000KN.
